# EUROPEAN PATENT APPLICATION

(11) **EP 3 936 078 A1**
(43) Date of publication of application: **12.01.2022**
(21) Application number: 20184424.8
(22) Date of filing: 07.07.2020
(51) Int. Cl.: A61B 42/10, A61B 42/50, A61B 42/40, A47G 25/90, B65D 83/08

(54) **GLOVE DONNING DEVICE AND GLOVE DEVICES FOR SAME**

(71) Applicant: Cosmo Machinery Co., Ltd., New Taipei City 23942 (TW)
(72) Inventor: LIN, Chun Liang, 23942 New Taipei City (TW)
(74) Representative: Viering, Jentschura & Partner mbB Patent- und Rechtsanwälte

(57) **Abstract**

A glove donning device includes a guide rack having a positioning portion, and a glove providing a connection portion at one side of the glove-wearing mouth thereof and a tear line at the junction of the glove and the connection portion. The connection portion is detachably fastened to the positioning portion of the guide rack to hand down the glove against one side of the guide rack. When the hand is putting on the glove, the guide rack holds the glove in place so that the glove will not move arbitrarily, and the user's hand can be easily and smoothly slip into the glove-wearing mouth and into the glove with a one-handed movement. Then, the hand is pulled outward to tear the tear line apart, so that the glove can be easily disconnected from the connection portion. This design solves the disadvantage that conventional gloves, such as thin-film gloves, must be put on with both hands.

## Description

### BACKGROUND OF THE INVENTION

### 1. Field of the Invention:

The present invention relates to glove technologies and, more particularly to a glove donning device, which allows the user to easily wear gloves without relying on each other's hands to assist the person wearing the gloves.

### 2. Description of the Related Art:

In many occasions of daily life, such as food processing (hand-made desserts, bread, pasta, etc.), health care, medicine preparation, kitchen cooking, housework, hair dyeing, barbecue, etc., you will wear gloves on your hands, especially when the hands come into contact with food, various objects or humans or animals, such as restaurants, restaurants, fresh markets, stores, medical institutions, etc. Mainly gloves or disposable gloves provide waterproof, antifouling, acid resistance, alkali resistance, isolation of bacteria and viruses, and avoid direct contact.

Among many gloves, disposable gloves such as PE film gloves have the advantages that the material is thin and the manufacturing cost is low, discarding after use can ensure the cleanliness and hygiene of the use, and the hand can still easily move after letting the user wear the gloves.

However, the flat thin-film gloves are soft and the inner and outer layers are attracted and adhered due to static electricity. The glove-wearing mouth on the outer layer is too tightly attached to the inner layer, which makes it difficult for the user to wear fingers into the glove-wearing mouth. Often the fingers cannot enter the glove-wearing mouth after twisted for a long time. When wearing, you must use one hand to help put the glove-wearing mouth of the glove onto the fingers of the other hand, and then slowly pull the glove to adjust it to cover the other hand. If you need to wear gloves on both hands, you must change hands to repeat the same wearing action and must spend one more time wearing. At the same time, if the hand is not clean, it is easy to touch the outside of the glove to cause contamination of the glove. Therefore, the problem of the convenience, speed, hygiene and anti-fouling of the glove is still not ideal.

### SUMMARY OF THE INVENTION

The present invention has been accomplished under the circumstances in view. It is the main object of the invention to provide a glove donning device, which includes gloves arranged on a guide rack so that the user can wear the glove on each hand without using both hands to help each other.

It is another object of the invention to provide a glove donning device, which is so configured that the user's fingers do not need to touch the outside of the glove, avoiding contamination on the outside of the glove.

It is still another object of the invention to provide a glove that allows the hand to wear it on and wear it off without the assistance of the other hand, including providing a plurality of gloves arranged in a stack, which can be continuously worn by multiple users.

According to some embodiments of the present invention, the gloves include thin-film gloves or general gloves, disposable thin-film gloves, gloves for use in medical hospitals, or isolation gloves for preventing bacteria or viruses, etc. The gloves may be selected from plastic, rubber, and silicone or any kind of paper materials.

To achieve the above-mentioned objects of the present invention, a glove donning device comprises a guide rack and at least one glove device. The guide rack comprises a bottom portion, a positioning portion extending a distance from the bottom portion, and at least one side surface portion located between the bottom portion and the positioning portion. Each glove device comprises a glove and a connection portion. The glove comprises an outer layer, an inner layer formed integral with the outer layer and connected to one side of the connection portion, a glove-wearing mouth formed between the free end of the outer layer and the inner layer, and a tear line provided at the junction of the connection portion and the inner layer. The connection portion is detachably coupled to the positioning portion so that the glove is supported on one side surface portion of the guide rack.

In some embodiments, the guide rack comprises a plurality of side surface portions respectively located in various directions between the bottom portion and the positioning portion.

In some embodiments, each glove device further comprises a second glove connected to an opposite side of the connection portion. The second glove comprises an outer layer and an inner layer, a glove-wearing mouth formed between the free end of the outer layer of the second glove and the inner layer of the second glove, the inner layer of the second glove being connected to the opposite side of the connection portion, and a tear line provided at the junction of the connection portion and the inner layer of the second glove. In this way, the two gloves are respectively connected to the two opposite sides of the connection portion, and two tear lines are respectively formed at the junctions of the two opposite sides of the connection portion and the two gloves. When the connection portion is detachably coupled to the positioning portion, the two gloves are hung down against the respective side surface portions. It is further known that a third or more gloves can be connected to the other sides of the connection portion and respectively hung down against the other side surface portions of the guide rack to achieve the same effect.

The invention also provides a glove device, which a glove and a connection portion. The glove comprises an outer layer, an inner layer formed integral with the outer layer and connected to one side of the connection portion, a glove-wearing mouth formed between the free end of the outer layer and the inner layer, and a tear line provided at the junction of the connection portion and the inner layer.

In some embodiments, the glove device further comprises a second glove connected to an opposite side of the connection portion. In this way, the two gloves are respectively connected to the two opposite sides of the connection portion, and two tear lines are respectively formed at the junctions of the two opposite sides of the connection portion and the two gloves.

When the hands are wearing the gloves, the guide rack holds the gloves in place so that the gloves will not move arbitrarily, making the left and right hands be easily and smoothly worn by the respective glove-wearing mouths into the respective gloves by one-handed movements. Then, the left and right hands are pulled outward to tear the respective tear lines apart, so that the gloves can be easily disconnected from the connection portion, which solves the disadvantage that conventional gloves (such as thin-film gloves) must be worn with both hands.

The present invention further provides disposable gloves that are convenient and quick to wear as a single use. The gloves also has the effects of waterproofing, antifouling, antibacterial, acid and alkali resistance, isolation and the like.

Other and further benefits, advantages and features of the present invention will be understood by reference to the following specification in conjunction with the accompanying drawings, in which like reference characters denote like elements of structure.

### BRIEF DESCRIPTION OF THE DRAWINGS

FIG. 1 is an oblique top elevational view of a glove donning device in accordance with the present invention.
FIG. 2 is an oblique top elevational view of gloves in accordance with the present invention.
FIG. 3 is a schematic drawing illustrating the assembly process of the gloves and the guide rack.
FIG. 4 is a front view of the gloves in accordance with the present invention.
FIG. 5 is a front view of another form of the gloves in accordance with the present invention.
FIG. 6 is a schematic drawing of the present invention, illustrating the glove wearing operation.
FIG. 7 is an elevational view of an alternate form of the glove donning device in accordance with the present invention.

### DETAILED DESCRIPTION OF THE PREFERRED EMBODIMENT

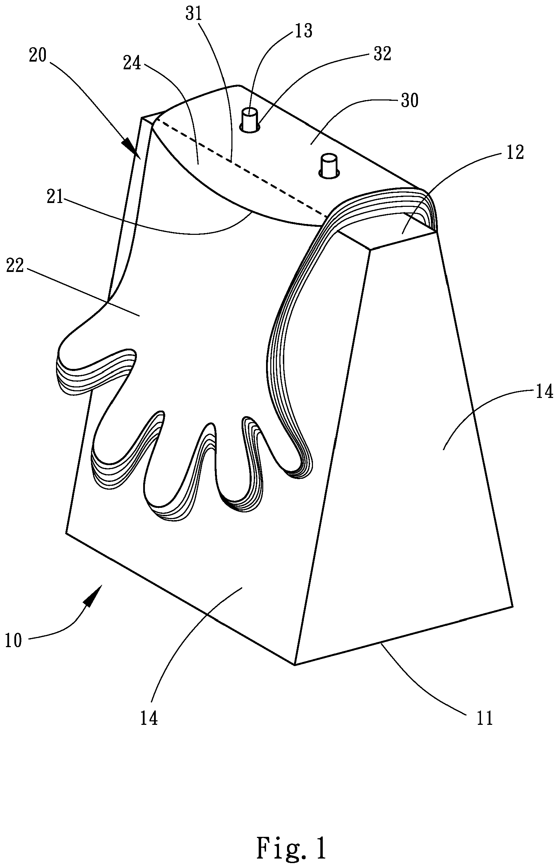

Referring to FIGS. 1-3, a glove donning device in accordance with the present invention is shown. The glove donning device comprises a guide rack **10** and at least one glove **20.**

In one structural embodiment, the glove donning device comprises a guide rack **10** and at least one glove **20.** The guide rack **10** comprises a bottom portion **11,** a positioning portion **12** extending a distance from the bottom portion **11,** and at least one, for example, a plurality of side surface portions **14** respectively located in different directions between the bottom portion **11** and the positioning portion **12.** The glove **20** comprises an outer layer **22** and an inner layer **24,** a glove-wearing mouth **21** formed between the free end of the outer layer **22** and the inner layer **24,** a connection portion **30** connected with one side thereof to the inner layer **24** at one side of the glove-wearing mouth **21,** and a tear line **31** provided at the junction of the connection portion **30** and the inner layer **24.** The connection portion **30** is detachably coupled to the positioning portion **12.** The glove **20** is arranged on one side surface portion **14** of the guide rack **10.** The guide rack **10** and the side surface portion **14** support the flat glove **20** to stand upright. The side surface portion **14** supports the outer side of the inner layer **24** of the glove **20.** According to this, when the hand performs the action of wearing the glove **20,** the positioning portion **12** holds the glove **20** in place, and the glove **20** can lean against the side surface portion **14,** so that the glove **20** can produce a fixed effect without arbitrarily moving. Thus, the action of the hand wearing the glove-wearing mouths **21** of the glove **20** is easier and smoother, the hand be easily and smoothly worn by the glove-wearing mouths **21** into the gloves **20** by one-handed movements. Then, the hand is pulled outward to tear the tear lines **31** apart, so that the gloves **20** can be easily disconnected from the connection portion **30,** which solves the disadvantage of that conventional gloves (such as thin-film gloves or paper-film gloved etc.) must be worn with both hands. In this manner users are able to wear the glove one-handed without the help of the other hand to wear the glove. In one structural embodiment, the tear line 31 includes, for example, perforated line, line of indentation, printed line of indentation, dotted-lined of indentation or line of the tooth mark etc. with tearing function that can be easily tear down the glove 20 to remove from the guide rack 10.

The glove donning device of the present invention is convenient for the user's hand to wear the glove **20** with a more ergonomic angle, for example, the hand can be used to open the glove-wearing mouth **21** in direction from top to bottom or from front to back using the fingertips. The positioning portion **12** holds the glove **20** in place. The side surface portion **14** supports against the glove **20,** and the user can smoothly insert the hand through the glove-wearing mouth **21** into the glove **20,** overcoming the softness problem of the flat thin-film glove and the adsorption and adhesion of the inner and outer layers, the docile problem of the glove-wearing mouth of the glove **20.**

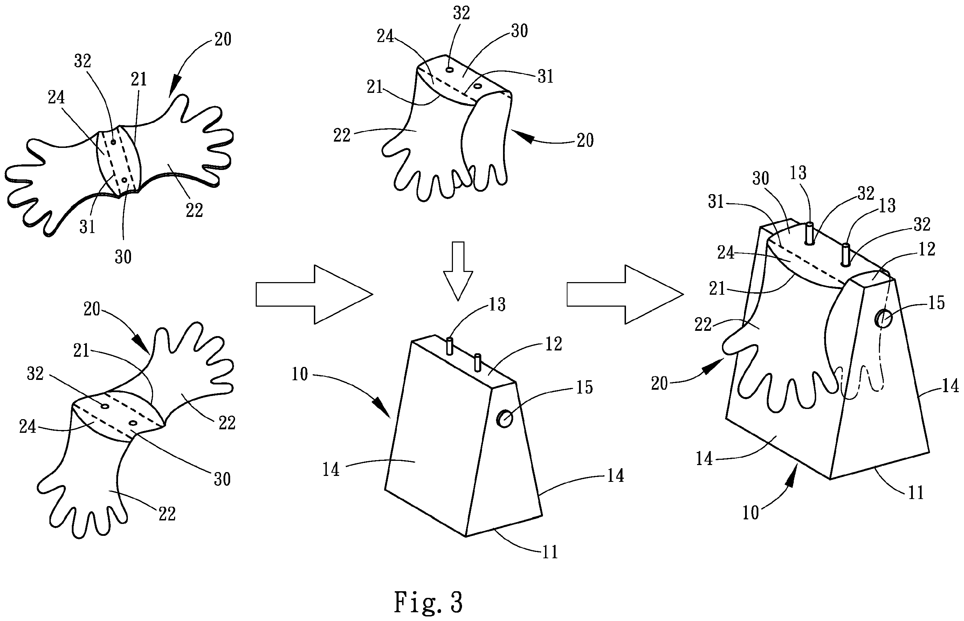

In one structural embodiment, as shown in FIGS. 1 and 3, the guide rack **10** comprises a plurality of side surface portions **14** located in various directions between the bottom portion **11** and the positioning portion **12** respectively.

The side surface portions **14** are, for example, inclined, parallel, or vertical. When the connection portion **30** is combined with the positioning portion **12,** the glove **20** can hang down against one of the side surface portions **14.** According to this, in some embodiments, the connection portion **30** is combined with a plurality of gloves **20,** for example, two, and the plurality of gloves **20** is connected to each side of the connection portion **30.** The plurality of gloves **20** is placed on the guide rack **10** and hangs down against different side surface portions **14,** respectively, and is used for wearing by multiple users.

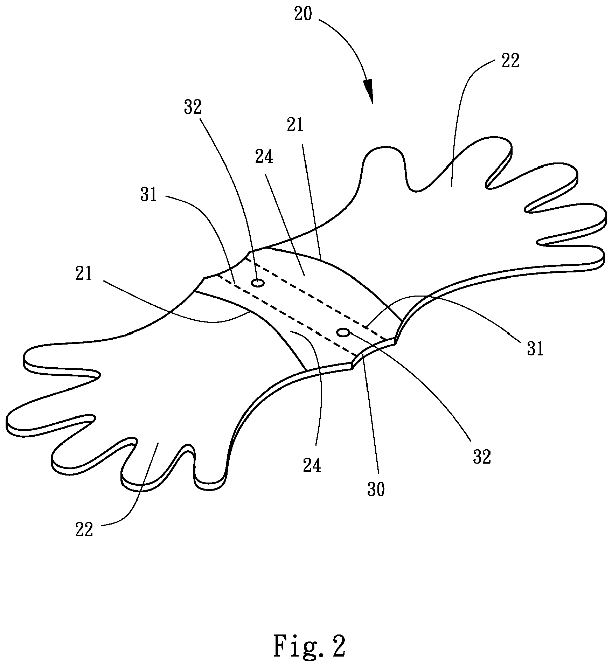

In one structural embodiment, as shown in FIGS. 2, 4 and 5, the glove donning device further comprises another glove **20** connected to an opposite side of the connection portion **30.** The said another glove **20** and the original glove **20** can be exactly the same structure or similar structure. The opposite side of the connection portion **30** is connected with the inner layer **24** at one side of the glove-wearing mouth **21** of the said another glove **20.** The connection portion **30** and the inner layer **24** of the said another glove **20** are set at a tear line **31.** In this way, the two gloves **20** are respectively connected to the two opposite sides of the connection portion **30,** and two tear lines **31** are formed at the junctions of the two connection portions **30** and the two gloves **20** respectively. When the connection portion **30** is detachably combined with the positioning portion **12,** the two gloves **20** can be hung down against the respective side surface portions **14.**

In one structural embodiment, the connection portion **30** has at least one perforation **32,** and the positioning portion **12** is provided with at least one protrusion **13.** The at least one positioning portion **12** is used to be attached onto the at least one protrusion **13** respectively.

In one structural embodiment, as shown in FIGS. 1 and 3, the guide rack **10** comprises two side surface portions **14** respectively located on left and right sides. In the present invention, the above two gloves **20** are respectively provided on both sides of the connection portion, and two tear lines **31** are separately formed at the junctions between the two sides of the connection portion **30** and the outer layers **22** of the two gloves **20,** respectively. The connection portion **30** further comprises two perforations **32.** The positioning portions **12** of the guide rack **10** is provided with two protrusions **13.** The two perforations **32** are used to be respectively attached onto the protrusions **13,** in order to place the gloves **20** on the guide rack **10** more stably. The two gloves **20** are respectively hung down against the two side surface portions **14** of the guide rack **10** so that the user can wear the gloves **20** on the two opposite sides of the guide rack **10** with the left and right hands. The gloves **20** are supported by the guide rack **10,** and the two hands can be smoothly slid from top to bottom to open the respective glove-wearing mouths **21** and extend into the two gloves **20,** the two hands (left and right hands) be easily and smoothly worn by the respective glove-wearing mouths **21** into the respective gloves **20** by one-handed movements. Then, the two hands (left and right hands) are pulled outward to tear the respective tear lines **31** apart, so that the gloves **20** can be easily disconnected from the connection portion **30,** which solves the disadvantage that conventional gloves (such as thin-film gloves) must be worn with both hands. In this manner.

In one structural embodiment, the protrusions **13** are, for example, columnar, vertical cylinders, and the perforation **32** are, for example, round holes, rectangular holes, or irregular holes.

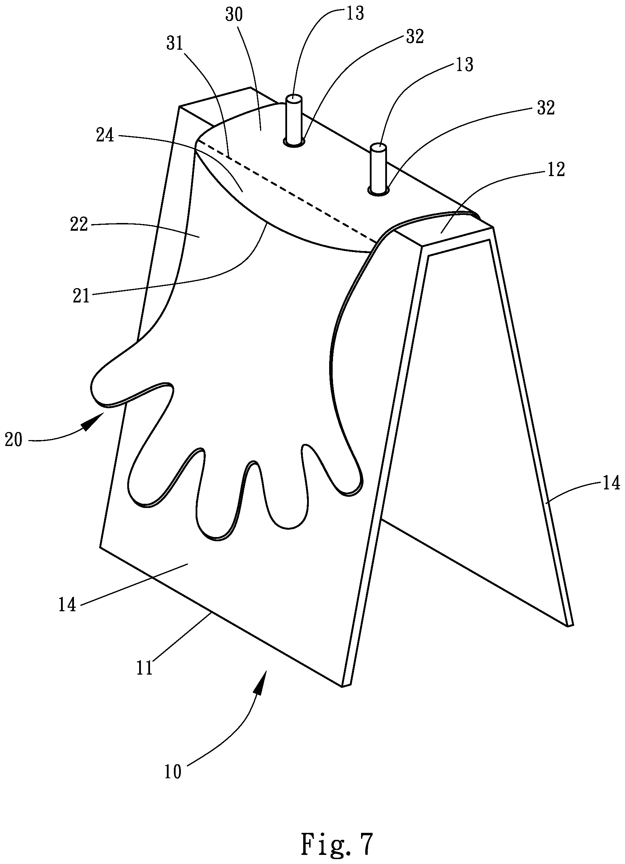

In one structural embodiment, as shown in FIG. 7, the guide rack **10** includes, but is not limited to, a metal frame, a metal plate, a wooden board, a plastic frame or a plastic board that is folded or molded or combined or injected with the bottom portion **11,** the positioning portion **12,** and the protrusions **13.** Its shape includes but is not limited to triangles or rectangles or arcs. Furthermore, the guide rack **10** can be a hollow structure or a solid structure. The bottom portion **11** or positioning portion **12** is, for example, planar or tripod. The connection portion **30** can be firmly positioned on the guide rack **10** to hang down the glove **20** at each side of the connection portion **30** against the respective side surface portion **14** for wearing by the user conveniently.

Referring to FIGS. 1 and 3, in one structural embodiment, the guide rack **10** is, for example, a plastic hollow member. The plastic hollow member can contain liquid such as water to increase the weight and have the effect of placing the center of gravity firmly on a flat surface.

The guide rack **10** can further be provided with a movable hole cover **15** that can be used for draining or refilling the liquid, and the weight is reduced when there is no liquid in the guide rack **10,** which can be easily moved or stored when not in use.

In one structural embodiment, as shown in FIGS. 1 and 3, the side surface portion **14** on each side of the guide rack **10** of the plastic hollow member, metal frame, metal plate, wooden board, plastic frame or plastic board listed above may be inclined, so that the area of the bottom portion **11** is wider, and the area of the positioning portion **12** is smaller than the bottom portion **11.** Preferably, the guide rack **10** forms a triangle-like shape structure, the bottom portion **11** or positioning portion **12** is a planar structure, in the embodiment is a rectangular planar structure.

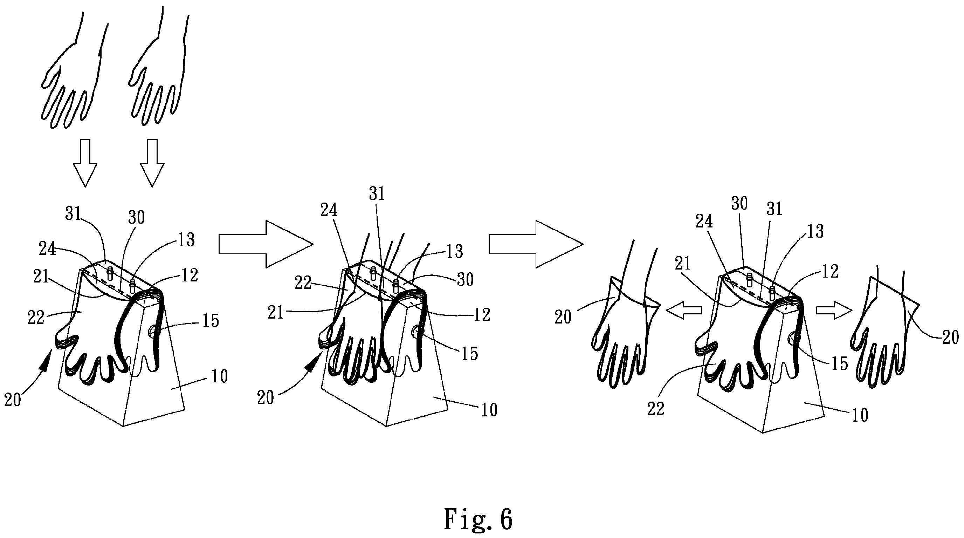

In one structural embodiment, as shown in FIGS. 1 and 6, the gloves **20** are stacked in a combined structure, and the gloves **20** are arranged on the guide rack **10,** so that the user or multiple users can continuously wear and use the gloves **20.**

When the glove **20** is used or discarded after being contaminated, the user can quickly and easily wear another glove **20** provided by the glove donning device of the present invention with one hand, to facilitate anti-fouling at work, isolate bacteria, viruses, etc., and maintain good sanitary condition.

In one structural embodiment, the guide rack **10** can be tilted in one direction to change the direction of the glove-wearing mouths **21** of the gloves **20,** such as up, left, right, front, and back, so that the hands can be extended from different directions into the glove-wearing mouths **21** while wearing the gloves **20.** It also allows users to choose the glove wearing direction that suits human factors or their own habits, improving the practicality of use and the inconvenience of wearing flat gloves.

In one structural embodiment, as shown in FIGS. 1-5, the glove **20** comprises an outer layer **22** and an inner layer **24,** a glove-wearing mouth **21** formed between the free end of the outer layer **22** and the inner layer **24,** a connection portion **30** connected with one side thereof to the inner layer **24** at one side of the glove-wearing mouth **21,** a tear line **31** provided at the junction of the connection portion **30** and the inner layer **24,** and at least one perforation **32** located on the connection portion **30.**

In one structural embodiment, the glove donning device further comprises another glove **20** connected to an opposite side of the connection portion **30.** The said another glove **20** and the original glove **20** can be exactly the same structure or similar structure. The opposite side of the connection portion **30** is connected with the inner layer **24** at one side of the glove-wearing mouth **21** of the said another glove **20.** The connection portion **30** and the inner layer **24** of the said another glove **20** are set at a tear line **31.** In this way, the two gloves **20** are respectively connected to the two opposite sides of the connection portion **30,** and two tear lines **31** are formed at the junctions of the two connection portions **30** and the two gloves **20** respectively. By this formation, there are two gloves **20** connected to the two sides of the connection portion **30** and two tear lines **31** are respectively formed at the junctions of the both sides of the connection portion **30** and the two gloves **20.**

In one structural embodiment, the connection portion **30** is provided with two perforations **32.** According to this, the gloves **20** are arranged at opposing left and right sides and the connection portion **30** with the gloves **20** can be placed on the guide rack **10** for use with both hands.

In one structural embodiment, the gloves **20** are selected from any one of PE, PP, PVC, plastic, silicone, paper or rubber materials.

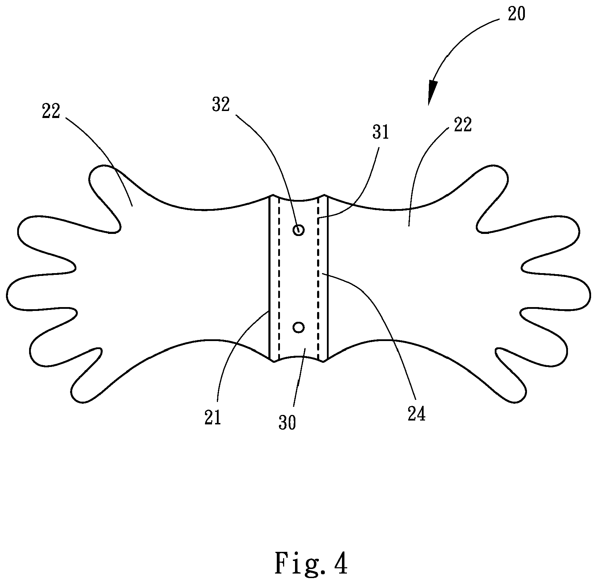

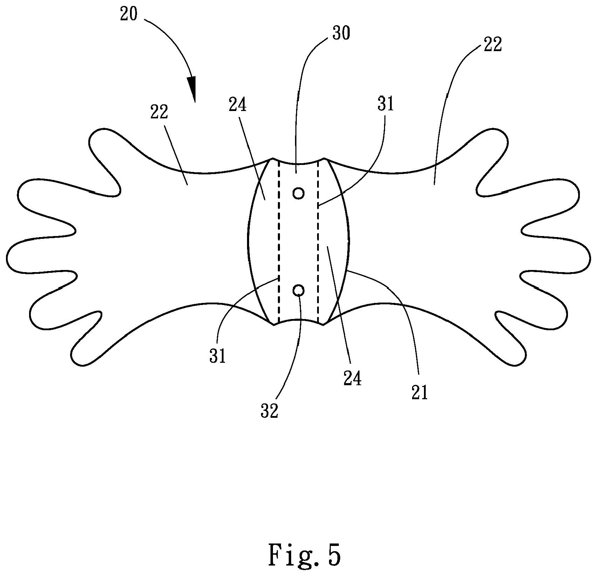

In one structural embodiment, as shown in FIG. 4, the glove-wearing mouth **21** is, for example, a linear slit opening. In one structural embodiment, as shown in FIG. 5, the glove-wearing mouth **21** can be made into a structure that is easier to open, such as a non-linear shape. The non-linear glove-wearing mouth **21** includes, for example, an arc-shaped slit opening, which can enlarge the opening of the glove-wearing mouth, or a tooth-shaped glove-wearing mouth, so that the hand can be easily and smoothly moved from glove-wearing mouth **21** to wear the glove **20.**

Referring to FIG. 3, the perforations **32** can be first set on the respective protrusions **13** of the positioning portion **12** to place the gloves **20** on the guide rack **10,** so that the gloves **20** form a hanging shape to lean against the respective side surface portions **14.**

Referring to FIGS. 1 and 6, the stacked gloves **20** are set on the protrusions **13** of the positioning portion **12** by means of the perforations **32** and hung down against the respective side surface portions **14.** Since the glove-wearing mouths **21** of the gloves **20** are facing upward, the hands can be directly extended into the glove-wearing mouths **21** from top to bottom, so that the palms of the hands can be worn in the gloves **20.** When the hands are wearing the respective gloves **20,** since the gloves **20** have the respective one sides positioned on the positioning portion **12,** the protrusions **13** and side surface portions **14** provide a resistive support force that can fix the gloves **20** so that the gloves **20** will not move arbitrarily, making the left and right hands be easily and smoothly worn by the respective glove-wearing mouths **21** into the respective gloves **20** by one-handed movements. Then, the left and right hands are pulled outward to tear the respective tear lines **31** apart, so that the gloves **20** can be easily disconnected from the connection portion **30,** which solves the disadvantage that conventional gloves (such as thin-film gloves) must be worn with both hands. In this manner, the next user can quickly use the gloves **20** of the present invention. When the gloves **20** arranged on the guide rack **10** are used up, the left connection portions can be removed from the positioning portion **12** of the guide rack **10** by means of the perforations **32** and then discarded or recycled, and a new stack of gloves **20** can be placed on the guide rack **10** for use.

The present invention further provides disposable gloves **20** that are convenient and quick to wear as a single use. The gloves **20** also has the effects of waterproofing, antifouling, antibacterial, acid and alkali resistance, isolation and the like.

Furthermore, the glove donning device of the present invention can be placed in restaurants, food and beverage stores, fresh markets, stores and other occasions where there are many consumers, so that each consumer can quickly and easily wear gloves **20,** so that the next consumer can also need not to wait and can wear gloves **20** immediately and conveniently. In this way, you can avoid the crowds of people in the queue caused by long-term waiting.

It is to be understood that the above-described embodiments of the invention are merely possible examples of implementations, merely set forth for a clear understanding of the principles of the invention, many modifications and enhancements may be made without departing from the spirit and scope of the invention. Accordingly, the invention is not to be limited except as by the appended claims.

## Claims

1. A glove donning device, comprising:
a guide rack comprising a bottom portion, a positioning portion extending a distance from said bottom portion, and at least one side surface portion located between said bottom portion and said positioning portion; and
at least one glove device, each said glove device comprising a glove and a connection portion, said glove comprising an outer layer, an inner layer formed integral with said outer layer and connected to one side of said connection portion, a glove-wearing mouth formed between the free end of said outer layer and said inner layer and a tear line provided at the junction of said connection portion and said inner layer, said connection portion being detachably coupled to said positioning portion so that said glove is supported on one said side surface portion of said guide rack.

2. The glove donning device as claimed in claim 1, wherein said guide rack comprises a plurality of side surface portions respectively located in different directions between said bottom portion and said positioning portion.

3. The glove donning device as claimed in claim 1 or 2, wherein each said glove device further comprises a second glove, said second glove comprising an outer layer and an inner layer, a glove-wearing mouth formed between the free end of the outer layer of said second glove and the inner layer of said second glove, the inner layer of said second glove being connected to an opposite side of said connection portion, and a tear line provided at the junction of said connection portion and the inner layer of said second glove.

4. The glove donning device as claimed in claim 1, wherein said connection portion is provided with at least one perforation; said positioning portion is provided with at least one protrusion for the positioning of said at least one perforation to hold said at least one glove device on said guide rack.

5. The glove donning device as claimed in claim 1 or 2, wherein said guide rack is selectively a metal frame, metal plate, wooden board, plastic frame or plastic board, or a plastic hollow member comprising said bottom portion, said positioning portion and said at least one side surface portion, and selectively made in a triangular, rectangular or arched shape.

6. The glove donning device as claimed in claim 5, wherein said at least one side surface portion of said guide rack is inclined so that the area of said bottom portion is wider, and the area of said positioning portion is smaller than said bottom portion.

7. The glove donning device as claimed in claim 1 or 2 or 3, wherein a plurality of said glove devices are arranged on said guide rack in a stack.

8. A glove device, comprising a glove and a connection portion, said glove comprising an outer layer, an inner layer formed integral with said outer layer and connected to one side of said connection portion, a glove-wearing mouth formed between the free end of said outer layer and said inner layer, and a tear line provided at the junction of said connection portion and said inner layer.

9. The glove device as claimed in claim 8, further comprising a second glove, said second glove comprising an outer layer and an inner layer, a glove-wearing mouth formed between the free end of the outer layer of said second glove and the inner layer of said second glove, the inner layer of said second glove being connected to an opposite side of said connection portion, and a tear line provided at the junction of said connection portion and the inner layer of said second glove.

10. The glove device as claimed in claim 8, wherein said connection portion is provided with at least one perforation.

11. The glove device as claimed in claim 8 or 9 or 10, wherein a plurality of said glove devices are arranged in a stack.

12. The glove device as claimed in any one of claims 8-11, wherein said glove-wearing mouth includes a linear slit opening or a non-linear slit opening.
